# EUROPEAN PATENT SPECIFICATION

(11) **EP 1 086 711 B1**
(45) Date of publication and mention of the grant of the patent: **10.12.2003**
(21) Application number: 00203264.7
(22) Date of filing: 20.09.2000
(51) Int. Cl.: A61L 27/44, A61L 27/46

(54) **Ceramic-polymer composites**
Keramic-Polymer-Zusammensetzungen
Matériaux composites céramique-polymère

(30) Priority: 24.09.1999 EP 99203141
(43) Date of publication of application: 28.03.2001
(73) Proprietor: IsoTis N.V., 3723 MB Bilthoven (NL)
(72) Inventor: Layrolle, Pierre Jean Francois, 3513 CT Utrecht (NL)
(74) Representative: Prins, Adrianus Willem

(56) References cited:
- WO-A-95/08304
- WO-A-99/19003
- US-A- 5 178 845
- US-A- 5 626 861
- US-A- 5 698 265
- DATABASE WPI Section Ch, Week 199941 Derwent Publications Ltd., London, GB; Class A96, AN 1999-481907 XP002131116 & JP 11 199209 A (KAGAKU GIJUTSUCHO MUKIZAISHITSU), 27 July 1999 (1999-07-27)
- DATABASE WPI Section Ch, Week 199830 Derwent Publications Ltd., London, GB; Class A96, AN 1998-341093 XP002131117 & JP 10 127753 A (AGENCY OF IND SCI & TECHNOLOGY), 19 May 1998 (1998-05-19)
- DUPRAZ A ET AL.: "INFLUENCE OF A CELLULOSIC ETHER CARRIER ON THE STRUCTURE OF BIPHASIC CALCIUM PHOSPHATE CERAMIC PARTICLES IN AN INJECTABLE COMPOSITE MATERIAL" BIOMATERIALS, vol. 20, no. 7, April 1999 (1999-04), pages 663-673, XP002131115

## Description

The invention relates to a method for preparing composite materials, based on ceramic and polymeric materials.

In bone replacement surgery, use is made of various materials. Implants for e.g. hips are often made of metallic materials, whereas cement fillers are usually polymeric in nature. Further, ongoing research takes place to manufacture implants of ceramic materials, such as hydroxyapatite, in order to more closely mimic the composition of natural bone.

In nature, however, many materials are based on polymer-ceramic composites, such as composites of collagen and hydroxyapatite in bone, chitin and calcium carbonate in shells. Therefore, several attempts have been made to prepare such composites. A problem encountered in such preparations, is that sintering steps, which are commonly employed in the preparation of ceramics, have to be avoided because the polymeric part of the composite would not be able to withstand the conditions necessary for sintering.

In order to study the properties of ceramic-polymer composites that occur in nature, Zhang and Gonsalves, Mat. Res. Soc. Symp., vol. 351, 1994, pp. 245-250, have prepared a calcium carbonate-chitosan composite, using low molecular weight polyacrylic acid as an additive. Crystal growth of calcium carbonate on a chitosan substrate was achieved from a supersaturated solution of calcium carbonate, at different concentrations of polyacrylic acid.

In the Journal of Materials Science: Materials in Medecin, vol. 4, 1993, pp. 58-65, Verheyen et al. have described an evaluation study of hydroxyapatite-poly(L-lactide) composites. The composites were prepared starting from granulated, sintered, crushed, milled and sieved hydroxyapatite (HA) and L-dilactide. To a mixture of these starting materials, stannous octoate was added in order to start polymerization to form the poly(L-lactide) (PLLA) in situ. Two different composites were prepared: one of PLLA with 0.30 wt.% HA, and one with 50 wt.% HA. As an alternative approach to the manufacture of the composite, plasma-spraying one side of an unfilled PLLA specimen with an HA coating was suggested. A disadvantage of the alternative approach is that it does not lead to a homogeneous composite, wherein the ceramic and polymer are uniformly distributed among each other. A disadvantage of the first method is that not all types of polymers can be polymerized in situ in the presence of a ceramic material. Also, the initiator used (stannous octoate) which is highly toxic will inevitably be incorporated into the composite.

The international patent application WO 99/19003 relates to implantable polymer/ceramic composites, the preparation of which involves the formation of a polymeric gel which is mixed with e.g. hydroxyapatite, and a sintering step at high temperature.

The Japanese patent application JP 11 199209 discloses the preparation of a composite material by coprecipitation of calcium phosphate and collagen, followed by pressing the obtained precipitate to form an organic-inorganic oriented composite material. The latter step is disadvantageous in that it involves relatively high pressures.

In the international patent application WO 95/08304 a method is disclosed for preparing a calcium phosphate cement by reacting an acidic phosphate powder with a basic calcium powder mixed with water and collagen. The chemical reaction between the acidic and basic powders leads to a cementing paste which may be hardened in vitro or in vivo. This method requires the use of specially formulated powders.

The present invention aims to provide a new method for preparing ceramic-polymer composites, which does not have the problems of the prior art methods for preparing this type of composite. A particular object of the invention is to provide a new method for preparing ceramic-polymer composites that may be used in replacement surgery.

Surprisingly, it has now been found that the above goals are reached by gel-precipitation and drying at low temperatures. Accordingly, the invention relates to a method for preparing a ceramic-polymer composite comprising the steps of precipitating a gel of the ceramic from an aqueous solution of the ceramic, mixing the gel with polymer, and drying at low temperature.

The method of the invention leads to a composite wherein the constituents are distributed among each other in a very homogeneous manner. Further, it is possible in a method according to the invention to prepare ceramic-polymer composites of various natures. Not only the chemical composition of both the ceramic and the polymeric constituent of the composite may be chosen broadly, also the ratio of the two constituent types can be chosen within a very broad range. Thus, the present invention provides a convenient, uniform method for preparing a ceramic-polymer composite of which the property profile may be set at will.

The present method mimics the natural way of making skeleton tissue, such as bones and teeth in vertebrates. In addition, the product to which the methods leads, mimics natural materials such as shells or exaskeletons of invertebrates. The organic component of the material, the polymer, provides a kind of backbone for crystal nucleation, growth and orientation. The mineral component, the ceramic, provides exceptional mechanical properties such as high compressive strength and hardness. In general, the organic component will preferably not exceed 30% in weight of the composite material. A very high strength may, for instance, be achieved in teeth composed of only 1 wt.% of proteins, in combination with submicrometer, highly oriented hydroxylcarbonate apatite crystals.

As the ceramic-polymer composite prepared in accordance with the invention is preferably used for applications in replacement surgery, it is preferred that it is biocompatible. This means that it is preferred that both the ceramic and the polymer on which the composite is based, are biocompatible. It is further preferred that the composite is biodegradable. Thus, it is also preferred that the ceramic and the polymer on which the composite based themselves are biodegradable.

In the context of the present invention, the term biocompatible is intended to refer to materials which may be incorporated into a human or animal body, e.g. in the form of a medical implant, substantially without unacceptable responses of the human or animal. The term biodegradable refers to materials which, after a certain period of time, are broken down in a biological environment. Preferably, the rate of breakdown is chosen similar or identical to the rate at which the body generates autogenous tissue to replace an implant of which the biodegradable material is manufactured.

Ceramic materials that are preferably used, are calcium phosphates and calcium carbonates, as these materials closely resemble the properties of natural bone materials. Highly preferred ceramic materials are octacalcium phosphate, apatites, such as hydroxyapatite and carbonate apatite, whitlockites, such as α-tricalcium phosphate and β-tricalcium phosphate, and combinations thereof.

The polymers used may be chosen from biological sources, such as polyaminoacids, silk, collagen, gelatin, casein, albumin, chitin, chitosan, polyhydroxyalkanoic acids, polysaccharides, cellulose, hyaluronic acid, alginates and alginic acid. Further, biodegradable or bioresorbable synthetic polymers may be used. Examples of such polymers are poly-L-lactic acid, polylysine, poly-D,L-lactic acid, polyglycolic acid, polydioxanone, polyorthoester, polycaprolactone, polycyanoacrylates or polyactive (a copolymer of a polyalkylene glycol, such as PEG, and a polyalkylteraphtalate, such as polybutylterephtalate). If a synthetic polymer is used, it will be chosen such that its degradation products, particularly in a biological environment, are essentially non-hazardous. Further, the polymer is preferably hydrophilic in nature. Highly preferred polymers are polyamino acids, which are preferably negatively charged.

Preferably, the polymer is incorporated in the freshly precipitated calcium phosphate gel in a fibrous, mesh or sponge form. This leads to a fiber reinforced ceramic having excellent properties after drying at room temperature. The polymers used for reinforcement should preferably interact with calcium phosphate or calcium carbonate ceramics. They should preferably be hydrophilic and negatively charged, carrying carboxyl, hydroxyl or phosphate groups. These polymers groups are bound or can complex/chelate calcium ions from the ceramic particles. The polymer further should preferably act as a binder for calcium phosphate or calcium carbonate ceramics. The polymers are preferably both biocompatible and biodegradable. Fibrous nonwoven meshes or polymeric sponges are particularly preferred.

In a preferred embodiment, the polymer can form a gel-like or colloidal substance in water at low concentration ( e.g. 2-10 wt %). This is the case with agarose, gelatine, alginic acid, polylactic or polyglycolic acids, hydroxylmethylcellulose. The polymer preferably has a very high molecular weight and hardens upon drying. The polymer fibers are preferably 5 to 50 microns in thickness, more preferably 5 to 20 microns, and 1 to 100 mm in length to form a sponge like or cotton like material.

In accordance with the present method, a gel of the ceramic is precipitated. Such a gel can suitably be formed in aqueous media by adding suitable amounts of salts, comprising the ions necessary for formation of the ceramic, to water. It is also possible to add to each other several aqueous solutions, each containing one or more of the ions necessary for formation of the ceramic.

In case, the ceramic is to be a calcium phosphate, which is preferred, an aqueous solution of a calcium salt may be mixed with an aqueous solution of a phosphate salts. Preferably, these salts are CaCl₂.6H₂O and Na₂HPO₄.12H₂O, respectively. It is preferred that the concentration of the calcium salt in the first solution lies between 0.05 and 1 M, more preferably between 0.1 and 0.8 M. The concentration of the phosphate salt preferably lies between 0.02 and 2 M, more preferably between 0.01 and 1.5 M. The ratio of the amount of the solution of the calcium salt to the amount of the solution of the phosphate salt (Ca/P ratio) preferably lies between 0.1 and 2, more preferably between 0.2 and 1. Preferably, both the calcium salt solution and the phosphate salt solution have a pH of 7 to 12, more preferably of 8 to 10. The desired pH may be set using for instance NaOH, KOH or NH₄OH.

In a preferred embodiment, one or more inhibitors of crystal growth are present in one or both of the solutions. Such an inhibitor may be a magnesium salt (Mg²⁺), a carbonate salt (HCO₃⁻), or a pyrophosphate salt (P₂O₇⁴⁻). Preferred inhibitors are magnesium chloride and sodium bicarbonate. Magnesium ions may be added to the calcium solution prior to mixing with the phosphate solution in an amount of from 1 to 50 mM, preferably from 1 to 10 mM. Carbonate and/or pyrophosphate ions may be added to the phosphate solution prior to mixing with the calcium solution in amounts of from 0.2 to 2 M, preferably from 0.5 to 1 M, and from 0.02 to 1 M, preferably from 0.01 to 0.5 M, respectively.

In a preferred embodiment, one or more bioactive compounds, such as a growth factor or a hormone, are added to one or both of the calcium and phosphate solutions. BMP's can for instance be incorporated into the composite to favor bone ingrowth. EGF or VEGF may be added to favor vascularization and blood vessel ingrowth. Fibronectin and related proteins may be added to favor cell attachment and colonization.

In another preferred embodiment, a pore maker or porogen compound is added to one or both of the calcium and phosphate solutions. In this regard, a pore maker compound is a compound that, during the drying and solidification of the gel, converts into or releases a gas, providing pores in the composite body. Open and interconnected pores having a size of 100 to 500 microns are preferred to facilitate cell integration, penetration, vascularization and biodegradation. The open porosity will permit bone ingrowth rather than sole bone contact from the edges of a dense body. Examples of suitable pore maker or porogen compounds are carbon dioxide gas and hydrogen peroxide. Carbon dioxide is a weak acid that could dissolve calcium phosphate crystals (freshly precipitated). During the mutual exchange of carbon dioxide gas with air, reprecipitation may occur leading to hardening of the composite. A non-toxic volatile organic solvent can also be used. The solvent may also be used to dissolve the polymer during mixing with the aqueous gel. During drying, the volatile compound will evaporate, forming pores in the composite body.

Upon mixing of the solutions, preferably with efficient stirring, a gel of the ceramic may precipitate. Preferably, this gel is left to mature for a period of time, e.g. between 0,5 and 5 hours. The pH is preferably neutral at this stage (between 6 and 8). In a preferred embodiment, the precipitation is carried out at room temperature without any further heat treatment. The gel-precipitate is dried at room temperature, i.e between 10 and 80°C, preferably between 20 and 50°C, with humidity control.

Subsequently, it is preferred to remove a large part of the water present in the gel. This may be accomplished by pouring the aqueous medium containing the gel on a filter, and allowing water to be removed, either by gravity alone or by the aid of applying vacuum. Care should be taken, however, that the gel is not dried to quickly, as this may lead to the formation of a cake, with cracks. Before this happens, the gel on the filter is preferably washed with water. It is also possible to remove part of the water by centrifugation. Preferably, the water content of the gel at this stage is between 250% and 750%, more preferably between 350% and 500%.

The thus obtained gel may suitably be mixed with the polymer. In case a polymer of a biological source is used, it may be added in solid form or dissolved in a suitable solvent. A suitable solvent preferably has a low boiling point, so that drying at room temperature or slightly elevated temperatures is feasible, and preferably is hydrophilic. A highly preferred solvent is water. Accordingly, water soluble polymers are preferred to ensure a homogeneous dispersion into the calcium phosphate gel. In case a synthetic polymer is used, it is added dissolved in a suitable solvent. Again, it is preferred that the polymer is hydrophilic and water soluble, so that it may act as binder for calcium phosphate particles. In general, it is also envisage to add monomers of the desired polymer to the gel and polymerize them in situ to form the polymer. The polymer will preferably be added to the gel in amounts of 1 to 40 wt.%, more preferably of 1 to 25 wt.%. At this stage, other additives may also be added. Examples of suitable additives include bio-active agents or drugs, such as growth factors, hormones, antibiotics and the like. The amounts of such additives should preferably not exceed such an amount as to disturb the formation of the ceramic-polymer composite.

The mixture of the gel and the polymer may subsequently be brought into a mold having the desired size shape. Preferably, the size of the mold is corrected for the slight degree of shrinkage that may occur during drying of the composite. Once the mixture is in the mold, it is desired to remove all gasses and excess water trapped inside the mixture, as these may lead to inhomogeneities and local loss of mechanical properties. Gasses may be removed by vibration and/or ultrasound.

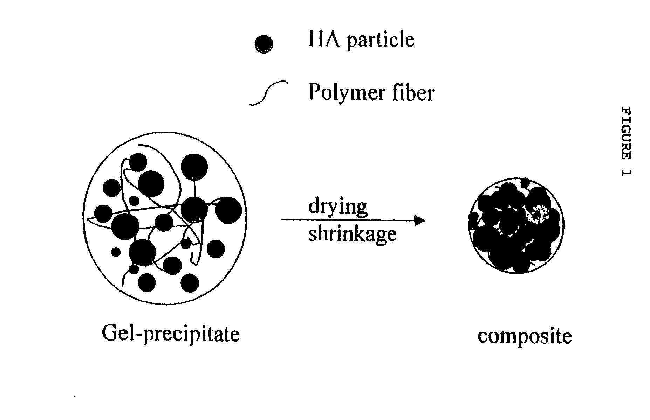

Finally, the composite is allowed to dry slowly in the mold at low temperature. It has been found that it is important not to let the composite dry to fast, as this may lead to shrinkage of an undesired extent and/or loss of mechanical properties. The attached figure schematically shows the drying of a gel-precipitate of a hydroxyapatite ceramic with polymeric fibers to form a composite.

Suitably, the composite may be dried at a temperature between 10 and 50°C, preferably between 15 and 40°C. Although it is possible to dry at reduced pressure, this is not preferred as this might increase the drying process too much. Also, the relative humidity during the drying is preferably less than 100%. More preferred is a relative humidity which slowly decreases during drying. Good results have been obtained by starting the drying process at a relative humidity between 90 and 70% and decreasing it to 10 to 5% in a period of 7-14 days. This procedure has been found to provide an optimum in drying rate and mechanical properties of the resulting composite. In order to regulate the drying process, a controlled decrease of relative humidity may be applied during drying. The final water content of the composite will be below 5 wt.%, preferably between 1 and 2 wt.%, based on the weight of the composite.

In a preferred embodiment, the mixture of the gel and the polymer comprises a weak gaseous acid, such as carbon dioxide, before drying. In case the polymer is added to the gel in the form of a solution or dispersion in water, this solution may be saturated with the gaseous acid. It is also possible to briefly bubble gaseous acid through the mixture of the gel and the polymer prior to drying. As a result, the pH of the mixture is decreased prior to drying and a slight dissolution of the ceramic present in the gel may take place. During the drying process, the gaseous acid will be released and the dissolved ceramic may reprecipitate. As by this time, the mixture may have shrunk a little, the reprecipitated ceramic may bind ceramic particles present in the mixture together, thus accelerating the drying process. It has been found that, particularly when the ceramic comprises calcium phosphate, the process benefits of this embodiment.

Depending on the composition of the composite, thus on the types of polymer and ceramic chosen to prepare the composite from, it may be used in various types of applications. In a preferred embodiment, the composite comprises calcium phosphate and is highly suitable to be used in replacement surgery, e.g. as bone filler, cement, implant, or scaffold for tissue engineering. In the latter case, the composite is preferably porous.

The invention will now be elucidated by the following, non-restrictive example.

### Example

Two solutions were prepared using the following ingredients:
A: 43.6 g CaCl₂.6H₂O (0.2 moles) , 1.25 g MgCl₂.6H₂O (0.006 moles), and 500 ml of demi water;
B: 218 g Na₂HPO₄.12H₂O (0.6 moles), 80 g NaHCo₃ (0.95 moles), 1500 ml demi water, and 80 ml of a 0.6 M solution of NH₄OH.

Solution A was stirred for 10 minutes, solution B was stirred for one hour. Next, solution A was poured quickly into solution B with rigorous stirring. A calcium phosphate gel was observe to precipitate. The obtained white, milky slurry was left to mature for 2 hours with stirring.

The slurry was next poured on a large funnel (20 cm), equipped with a whatmann filter paper disc #2. Vacuum was applied to remove water. Before the gel on the funnel was formed into a cake with cracks, it was rinsed with 500 ml of demi water. The vacuum was stopped and the wet gel was moved with a spatula into a 100 ml beaker.

To this beaker, 50 ml of demi water was added, as well as 50 ml of a colloidal solution of agarose (8 wt.%) in demineralized water. The mixture was stirred with a spatula until a yogurt-like gel was obtained. This was poured into a teflon mold, which was vibrated for 15 minutes to remove trapped air bubbles. Further degassing was performed using ultrasound.

Next, the mold was put into a climatic chamber which was maintained at a constant temperature of 37°C. The relative humidity in the chamber was reduced slowly from 90% to 40%. After two weeks, a dry composite was removed from the mold.

## Claims

1. A method for preparing a ceramic-polymer composite comprising the steps of precipitating a gel of the ceramic from an aqueous solution of the ceramic, mixing the gel with polymer and drying at low temperature.

2. A method according to claim 1, wherein the ceramic is a calcium phosphate or calcium carbonate.

3. A method according to claim 1 or 2, wherein the polymer is fibrous.

4. A method according to any of the preceding claims, wherein the polymer is chosen from the group of biocompatible and biodegradable polymers.

5. A method according to claim 4, wherein the polymer is chosen from the group of polyvinyl alcohol, polysaccharides, proteins, alginates, polylactides, polyglycolides, copolymers of a polyalkylene glycol and an aromatic ester, and combinations thereof.

6. A method according to any of the claims 2-5, wherein the gel is prepared by mixing a first solution comprising calcium ions, and a second solution comprising phosphate ions.

7. A method according to claim 6, wherein one of the first and second solutions comprises magnesium ions.

8. A method according to claim 6 or 7, wherein one of the first and second solutions comprises bicarbonate ions.

9. A method according to any of the preceding claims, wherein the drying is carried out at a temperature between 10 and 50°C, preferably between 15 and 40°C.

10. A method according to any of the preceding claims, wherein the drying is carried out at a relative humidity of less than 100%.

11. A ceramic-polymer composite obtainable by a method according to any of the preceding claims.

12. The use of the ceramic-polymer composite according to claim 11 for the manufacture of a preparation for use in replacement surgery.

## Patentansprüche

1. Verfahren zur Herstellung eines Keramik/Polymer-Verbundmaterials, umfassend die Schritte des Präzipitierens eines Gels des Keramikmaterials aus einer wässrigen Lösung des Keramikmaterials, Mischen des Gels mit Polymer und Trocknen bei niedriger Temperatur.

2. Verfahren gemäß Anspruch 1, worin das Keramikmaterial ein Calciumphosphat oder Calciumcarbonat ist.

3. Verfahren gemäß Anspruch 1 oder 2, worin das Polymer faserig ist.

4. Verfahren gemäß einem der vorstehenden Ansprüche, worin das Polymer ausgewählt ist aus der Gruppe biokompatibler und biologisch abbaubarer Polymere.

5. Verfahren gemäß Anspruch 4, worin das Polymer ausgewählt ist aus der Gruppe von Polyvinylalkohol, Polysacchariden, Proteinen, Alginaten, Polylactiden, Polyglykoliden, Copolymeren eines Polyalkylenglykols und eines aromatischen Esters und Kombinationen derselben.

6. Verfahren gemäß einem der Ansprüche 2 bis 5, worin das Gel durch Mischen einer ersten Lösung, enthaltend Calciumionen, und einer zweiten Lösung, enthaltend Phosphationen, hergestellt wird.

7. Verfahren gemäß Anspruch 6, worin eine der ersten und zweiten Lösungen Magnesiumionen umfasst.

8. Verfahren gemäß Anspruch 6 oder 7, worin eine der ersten und zweiten Lösungen Bicarbonationen umfasst.

9. Verfahren gemäß einem der vorstehenden Ansprüche, worin das Trocknen bei einer Temperatur zwischen 10 und 50°C, vorzugsweise zwischen 15 und 40°C erfolgt.

10. Verfahren gemäß einem der vorstehenden Ansprüche, worin das Trocknen bei einer relativen Feuchtigkeit von weniger 100 % erfolgt.

11. Keramik/Polymer-Verbundmaterial, erhältlich über ein Verfahren nach einem der vorstehenden Ansprüche.

12. Verwendung des Keramik/Polymer-Verbundmaterials gemäß Anspruch 11 bei der Herstellung einer Zubereitung zur Verwendung in der Austauschchirurgie.

## Revendications

1. Un procédé de préparation d'un composite de ceramique-polymère comprenant les étapes de précipitation d'un gel de céramique à partir d'une solution aqueuse de la céramique, de mélange du gel avec un polymère et de sechage à basse temperature.

2. Une procédé selon la revendication 1, dans laquelle la céramique est un phosphate de calcium ou un carbonate de calcium.

3. Un procédé selon les revendications 1 ou 2, dans laquelle le polymère est fibreux.

4. Un procédé selon les revendications précédentes, dans laquelle le polymère est choisi parmi le groupe des polymères biocompatibles ou biodégradables.

5. Un procédé selon la revendication 4, dans laquelle le polymère est choisi parmi le groupe de l'alcool polyvinylique, polysaccharoses, protéines, alginates, polylactides, polyglycolides, les copolymères d'un polyalkylène glycol et un ester aromatique, et une combinaisons de ceux-ci.

6. Un procédé selon l'une quelconque des revendications 2-5, dans laquelle le gel est préparé en mélangeant une première solution comprenant des ions calcium, et une seconde solution comprenant des ions phosphate.

7. Un procédé selon la revendication 6, dans laquelle une de la première ou deuxième solution comprend des ions magnésium.

8. Un procédé selon les revendications 6 ou 7, dans laquelle une de la première et deuxième solution comprend des ions bicarbonate.

9. Un procédé selon l'une quelconque des revendications précédentes, dans laquelle le séchage est effectué à une température entre 10 et 50°C, de préférence entre 15 et 40°C.

10. Un procédé selon l'une quelconque des revendications précédentes dans laquelle le séchage est effectué à une humidité relative inférieure à 100%.

11. Un composite céramique-polymère susceptible d'être obtenu par une méthode selon l'une quelconque des revendications précédentes.

12. L'utilisation du composite céramique-polymère selon la revendication 11 pour la fabrication d'une préparation pour utilisation dans la chirurgie de remplacement.
